(19) **Europäisches Patentamt**

**European Patent Office**

**Office européen des brevets**

(11) **EP 0 635 722 B1**

(12) # EUROPEAN PATENT SPECIFICATION

(45) Date of publication and mention
of the grant of the patent:
**26.05.1999 Bulletin 1999/21**

(51) Int Cl.6: **G01N 33/74**

(21) Application number: **94202118.9**

(22) Date of filing: **19.07.1994**

(54) **Antenatal screening for chromosomal abnormalities**

Vorgeburtliche Überprüfung von Missbildungen der Chromosomen

Dépistage prénatal des anomalies chromosomiques

(84) Designated Contracting States:
**AT BE CH DE DK FR GB IT LI NL SE**

(30) Priority: **22.07.1993 GB 9315230**

(43) Date of publication of application:
**25.01.1995 Bulletin 1995/04**

(73) Proprietors:
• **KODAK LIMITED**
  **Harrow, Middlesex HA1 4TY (GB)**
  Designated Contracting States:
  **GB**
• **Johnson & Johnson Clinical Diagnostics, Inc.**
  **Rochester New York 14650 (US)**
  Designated Contracting States:
  **BE CH DE DK FR IT LI NL SE AT**

(72) Inventor: **Davies, Christopher John,**
**Kodak Limited**
**Harrow, Middlesex, HA1 4TY (GB)**

(74) Representative: **Mercer, Christopher Paul et al**
**Carpmaels & Ransford**
**43, Bloomsbury Square**
**London WC1A 2RA (GB)**

(56) References cited:
**WO-A-89/00696**     **WO-A-90/08325**
**WO-A-94/12884**     **US-A- 4 454 232**
**US-A- 4 874 693**

• **British Medical Journal, 297, 1988, 883-887**
• **British Journal of Obstetrics and Gynaecology, 94, 1987, 387-402**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to a method for antenatal screening for chromosomal abnormalities and to an apparatus for performing the method and in particular to a method and apparatus for antenatal screening for Down's Syndrome.

The risk of Down's Syndrome and some other chromosomal abnormalities in a foetus is known to increase with the age of the mother and it is this knowledge which forms the basis for selection of pregnant women for further investigation. Further investigation for instance in the case of Down's Syndrome involves sampling of the amniotic fluid by amniocentesis, a procedure which itself carries a risk for the mother of the foetus, induction of a miscarriage being a recognised hazard of this procedure.

Maternal serum and other markers for Down's Syndrome are widely used for antenatal screening for this chromosomal abnormality, the most common of these markers being alpha-fetoprotein (AFP), human chorionic gonadotropin (hCG) - either the intact molecule thereof or its beta subunit - and unconjugated estriol (UE). Disclosures relating to the use of these markers in antenatal screening for Down's Syndrome include US Patent 4,874,693, WO 89/00696 and WO 90/08325.

Maternal serum screening is based on selecting a subgroup of women who are at the greatest risk of giving birth to a child with an abnormality, in whom the risks of an invasive diagnostic procedure are considered to be outweighed by the risk of the abnormality. The risk is calculated by multiplying the a priori age related risk by the likelihood ratio. The likelihood ratio is calculated from the relative frequencies of the multivariate Gaussian distribution functions of marker analytes in affected and unaffected pregnancies, corresponding to the value of the individual serum marker concentrations.

Since the concentrations of the various analytes vary with gestational age, the analyte concentrations must be normalised. This is performed by dividing the concentration of the analyte by the median concentration expected for that particular gestational age in women with unaffected pregnancies. This is termed the multiple of the median (MoM).

The use of two or more markers together in antenatal screening can be advantageous. For example the markers AFP, hCG and UE can be used together. The combination of marker analytes gives significantly more information than is given by any single marker alone, or by the group of markers when used sequentially. The use of likelihood ratios derived from a multivariate combination is an efficient means of deriving information relating to a woman's risk of carrying an affected child.

Hitherto it has generally been assumed that information relating to a woman's risk of carrying an unaffected child which is derived from markers such as AFP, hCG and UE is independent of maternal age. We believe that this assumption is correct with AFP and hCG and in many other instances. However in some instances, and particularly with UE, the assumption is not correct and the information relating to risk is influenced by maternal age. Therefore when using markers such as UE allowance should be made for maternal age when processing any information which is obtained.

According to the present invention we provide a method for antenatal screening for chromosomal abnormalities in which a maternal body fluid from a pregnant woman is measured for the level of at least one marker and/or a precursor or metabolite of said marker and the measured level of this marker together with the gestational age of the pregnant woman are compared to reference values at various gestational ages of the level for the marker in (a) pregnant women carrying foetuses having the abnormality(ies) subject to the screen and (b) pregnant women carrying normal foetuses, the comparison being indicative of the risk of the pregnant woman carrying a foetus with an abnormality subject to the screen characterised in that the level of the marker is affected by maternal age and in making the comparison allowance is made for the age of the pregnant woman.

Further according to the present invention we provide an apparatus comprising means adapted for receiving measurements of a pregnant woman's maternal body fluid level of at least one marker and/or a precursor or metabolite of said marker and computer means for comparing the measurements of this level to sets of reference data to determine fetal chromosomal abnormalities characterised in that the level of the marker is affected by maternal age and the apparatus comprises means for adjusting the marker level measurements to take account of this.

The maternal body fluids on which measurements are made include for example saliva, urine, amniotic fluid and particularly blood. Preferably the marker is a serum or plasma marker.

The method of the invention can be used for antenatal screening for a wide range of chromosomal abnormalities. The most significant and frequently occurring of these is Down's Syndrome (Trisomy 21). Other abnormalities which may be screened for using the invention include Edwards Syndrome (Trisomy 18), Pateaus Syndrome (Trisomy 13), Turner Syndrome, Monosomy X and Kleinefelter's Syndrome. The method of the invention may be used to screen for individual abnormalities or to screen for groups of abnormalities together, for example it could be used to screen for both Down's Syndrome and Edwards Syndrome.

Markers whose levels in a women's blood are affected by age include dehydroepiandrosterone sulphate (DHEAS), 16-alpha-hydroxy-dehydroepiandrosterone sulphate (16-alpha-hydroxy DHEAS) and, particularly (UE) and precursors and/or metabolites of these.

The method of the invention can be used to analyse risk using other serum markers in blood samples in combination with the age-affected marker. Such other markers include alpha-fetoprotein (AFP), inhibin (In), progesterone (Pr), human chorionic gonadotropin (hCG) - either the intact molecular or its Beta subunit, pregnancy associated plasma protein A (PAPPA), and precursors and metabolites of these markers and UE, DHEAS and 16-alpha-hydroxy-DHEAS if not already included. Thus one or more of these other markers may be measured together with the principal markers to be measured by the method of the invention. For example the method of the invention may be used with hCG, PAPPA and UE as the markers to be measured.

Measurements are carried out and analysed using the method of the invention on blood samples taken during an appropriate period of pregnancy. Preferably the measurements are made on blood samples taken in the first trimester and especially in the period between the beginning of the eighth week and the end of the thirteenth week of gestation (8th to 13th weeks). The woman's measured serum value for the individual serum marker is divided by the expected median value found in women with unaffected pregnancies at the same gestational age, to derive the (MoM). The probability that the (MoM) values for the combination of serum markers tested belongs to the multivariate distribution of values found in unaffected pregnancies is calculated. The same calculation is performed by reference to the probability that the individual combination of values forms part of the multivariate distribution found in abnormal pregnancies. The ratio of the two probabilities is termed the likelihood ratio (LR) which indicates the likelihood that an individual woman has an affected pregnancy or not. The degree of separation between the multivariate distributions for affected and unaffected pregnancies changes with gestational age, i.e. there is a continuous change in the manner of calculating probability depending upon the gestational age. This continuous change can be built into the algorithm used in the calculation.

An individual woman has an a priori age related risk which is independent of the maternal serum marker concentrations. The woman's age related risk, by Baye's theorum, is modified by multiplying by the likelihood ratio (LR) obtained previously to derive a combined risk. This combined risk may then be used to counsel the woman regarding the relative risk of the abnormality as opposed to the risk of miscarriage associated with a subsequent diagnostic invasive procedure.

The invention is illustrated by the accompanying drawings wherein:-

Figure 1 is a diagram of frequency against AFP MoM (log scale);
Figure 2 is a diagram of frequency against hCG MoM (log scale);
Figure 3 is a diagram of frequency against UE MoM (log scale);
Figure 4 is a plot of UE MoM (log scale) against maternal age at date of delivery for data from Trials A in Example 1;
Figure 5 is a plot of AFP MoM (log scale) against maternal age at date of delivery for data from Trials A in Example 1;
Figure 6 is a plot of hCG MoM (log scale) against maternal age at date of delivery for data from Trials A in Example 1;
Figure 7 is a plot of UE MoM (log scale) against maternal age at date of delivery for data from Trials B in Example 1;
Figure 8 is a plot of AFP MoM (log scale) against maternal age at date of delivery for data from Trials B in Example 1;
Figure 9 is a plot of hCG MoM (log scale) against maternal age at date of delivery for data from Trials B in Example 1;
Figure 10 is a plot of UE MoM (log scale) against maternal age at date of delivery for combined data from Trials A and B in Example 1;
Figure 11 is a plot of AFP MoM (log scale) against maternal age at date of delivery for combined data from Trials A and B in Example 1; and
Figure 12 is a plot of hCG MoM (log scale) against maternal age at date of delivery for combined data from Trials A and B in Example 1.

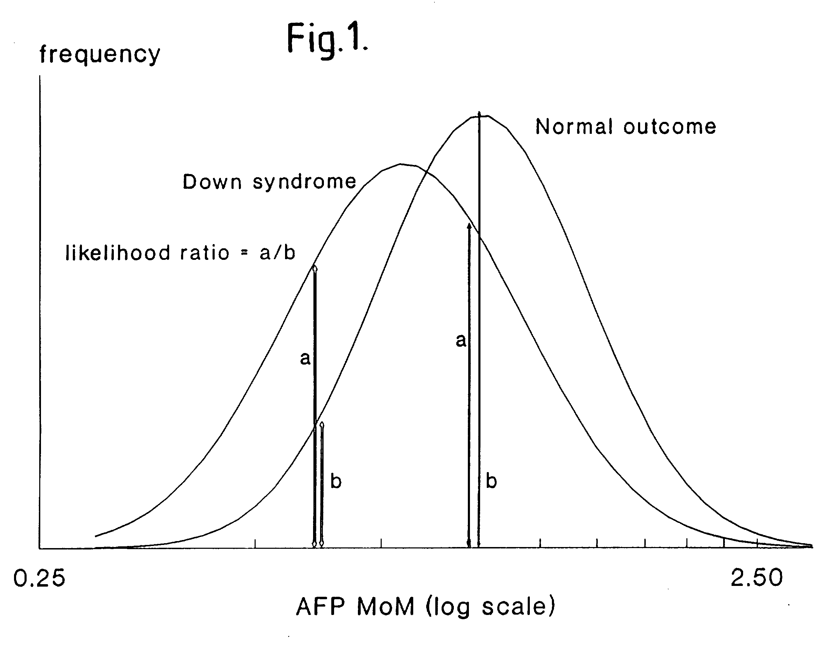

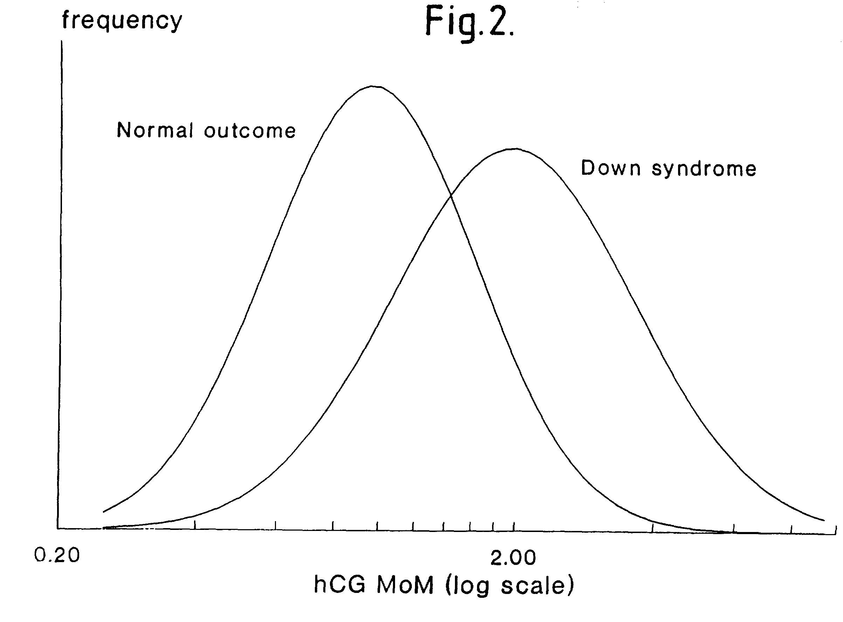

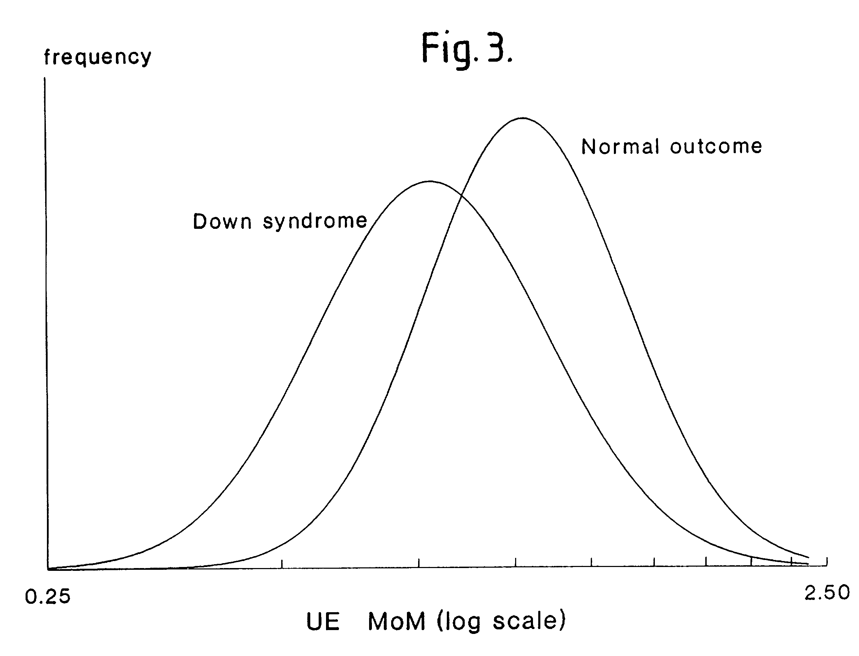

Figure 1 demonstrates the principles involved in calculating the <u>likelihood</u> that a sample came from a Down Syndrome affected pregnancy, in this case for maternal serum alpha-fetoprotein (AFP), although the same principle applies to human chorionic gonadotrophin (hCG), unconjugated estriol (UE) or indeed any marker, biochemical or otherwise in which the distributions in affected and unaffected pregnancies differ. The distributions in affected and unaffected pregnancies for hCG and UE are shown in Figures 2 and 3 respectively.

The invention is further illustrated by the following examples:-

Example 1

The relationship between maternal age and maternal serum unconjugated estriol.

Two large databases were examined for a relationship between maternal serum concentrations of biochemical markers and maternal age. The first (Trials A) was a set of 2545 women with normal outcome pregnancies. The data came from 5 sites throughout Europe. The second set of data (Trials B) consisted of 3124 women from a single laboratory in the United Kingdom who were being screened for neural tube defects. All samples were assayed for maternal serum alpha-fetoprotein (AFP), human chorionic gonadotrophin (hCG), and unconjugated estriol (UE), using Kodak Amerlex-M second trimester assays according to the manufacturers' (Kodak Clinical Diagnostics Ltd., Amersham, UK) instructions. (N.B. KODAK and AMERLEX are Trademarks). For each trial centre median values were established at each week of normal gestation from 15 to 20 completed weeks of pregnancy. A regression equation was calculated for each analyte relating the median analyte value to the numbers of days of gestation. AFP and UE showed a logarithmic increase with gestational age, whereas hCG showed an exponential decline over the weeks studied. Separate regressions were established for each study centre.

Each sample result was then converted to the multiple of the median value by dividing by the expected median found at that gestational age.

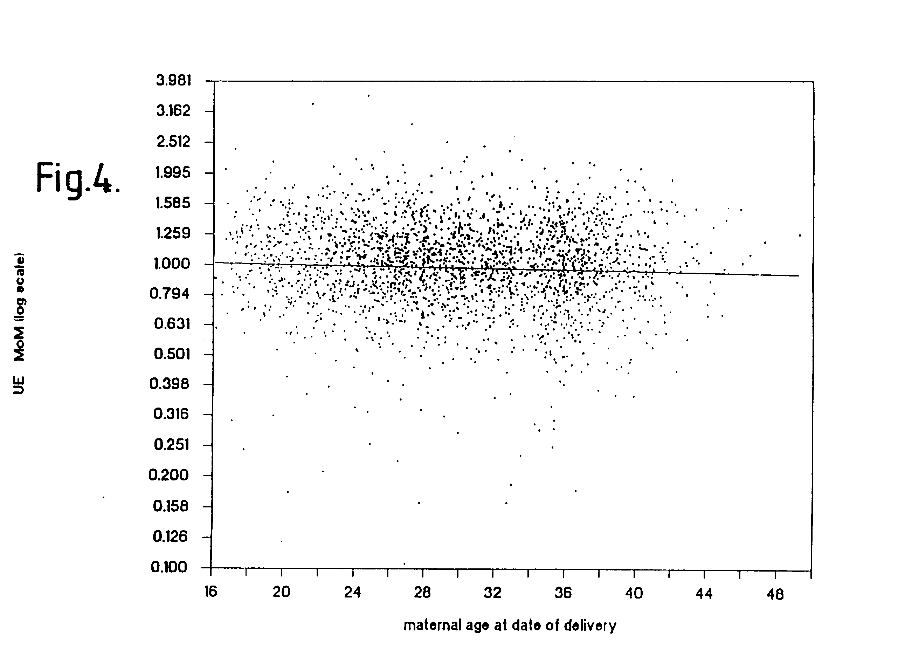

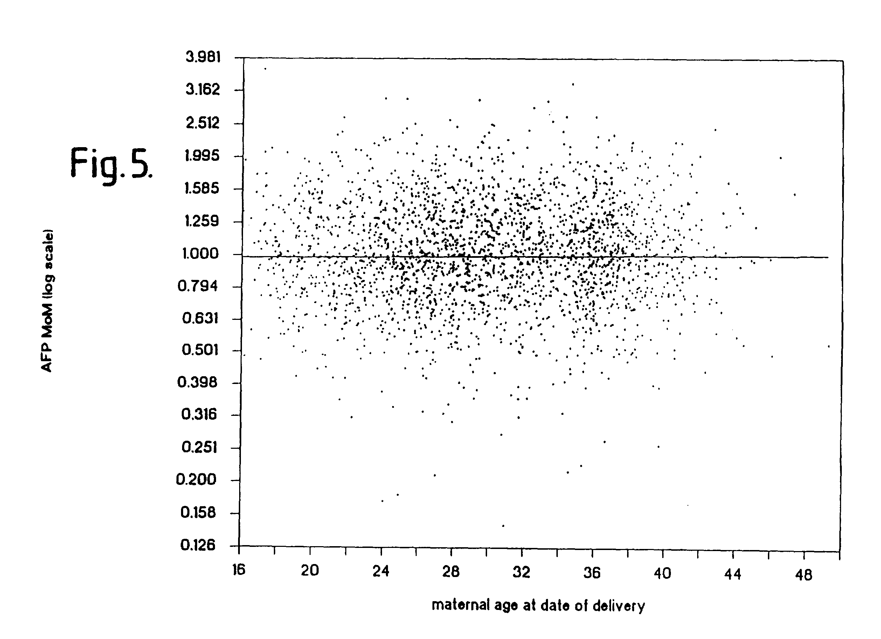

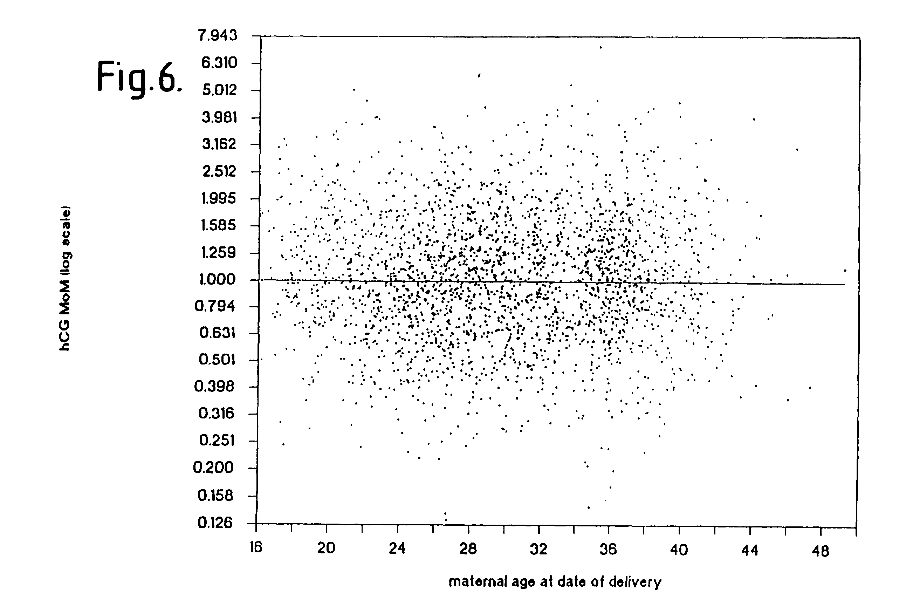

Figure 4 shows the relationship between unconjugated estriol and maternal age at the expected date of delivery for data from Trials A. There is a highly statistically significant relationship with unconjugated estriol being lower in older women than in younger. The relationship was best described by regression of the natural log of the MoM value against maternal age at the expected date of delivery, as all three analytes (AFP, hCG, UE) were found to follow a log Gaussian distribution. No relationship was found in these women between maternal age and either maternal serum AFP MoM or maternal serum hCG MoM (Figures 5 and 6 respectively)

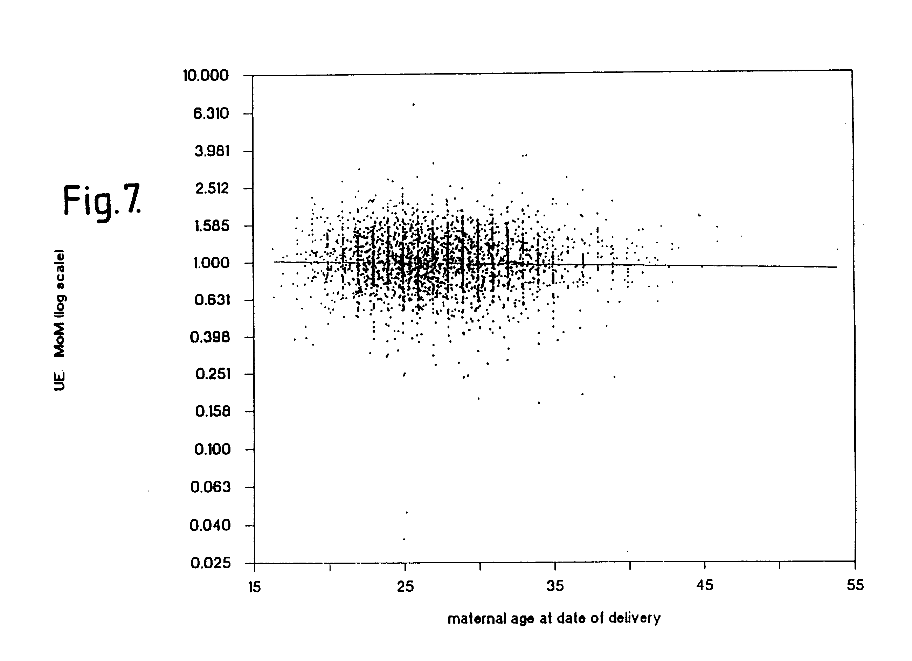

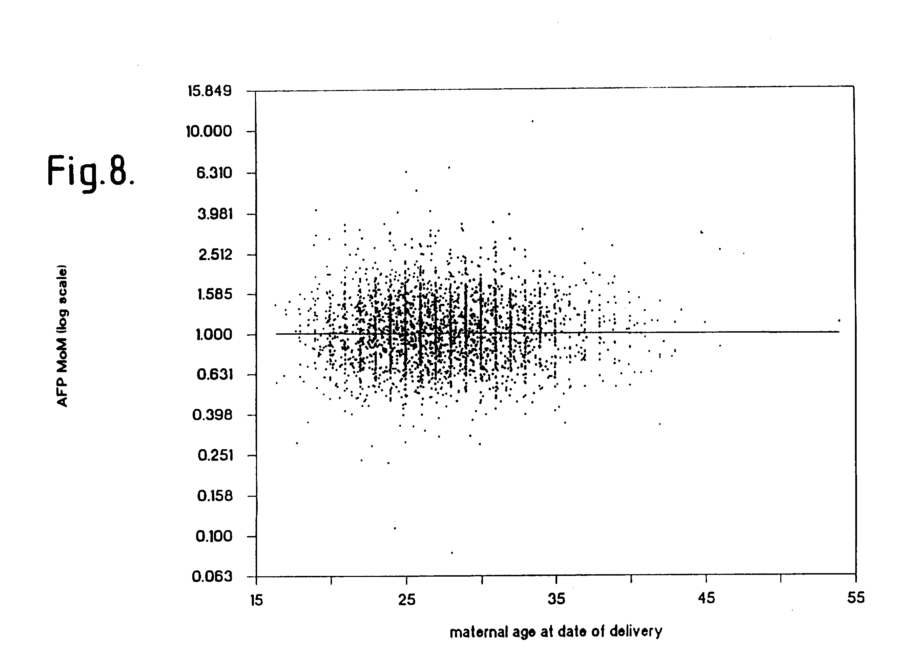

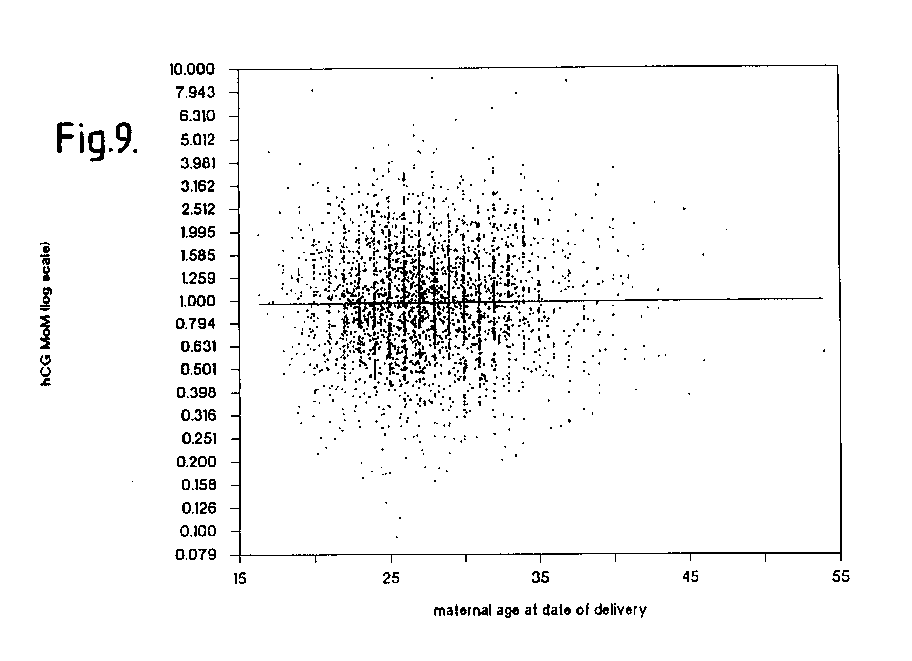

Examination of data from Trials B showed a strikingly similar effect, shown in Figure 7. Again no relationship was found between maternal serum AFP MoM or hCG MoM and maternal age as shown in Figures 8 and 9 respectively.

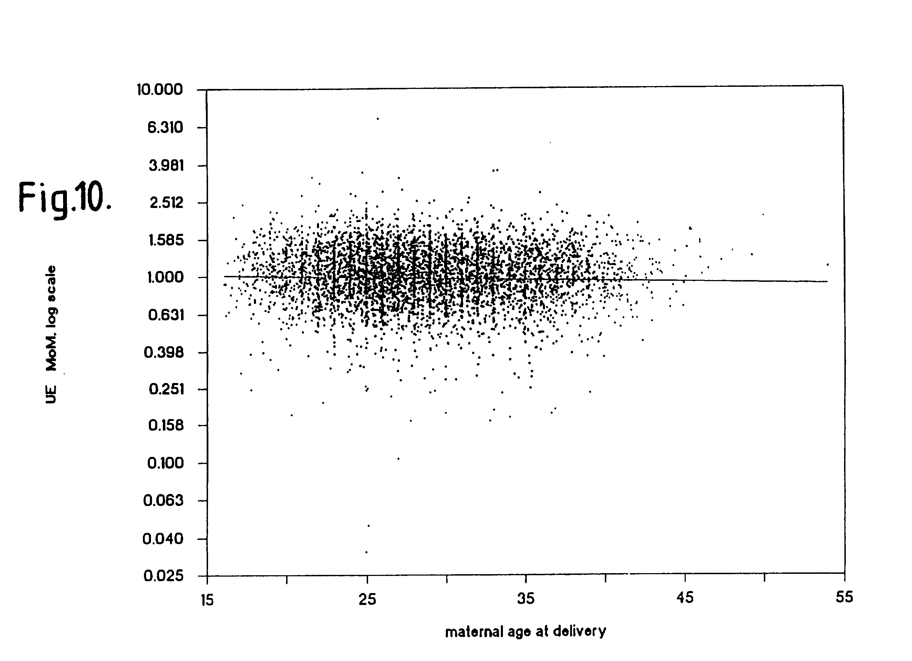

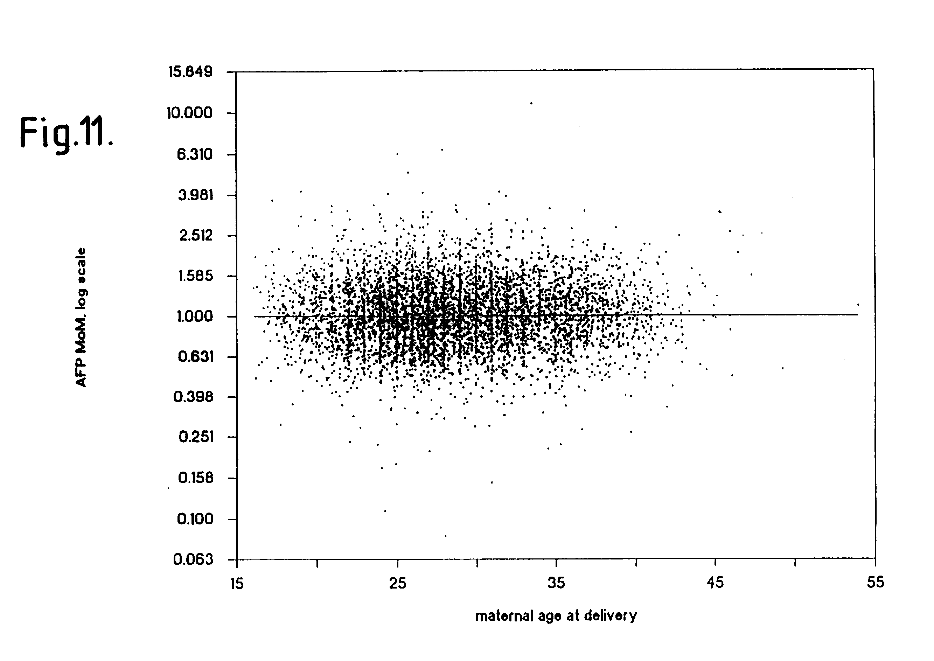

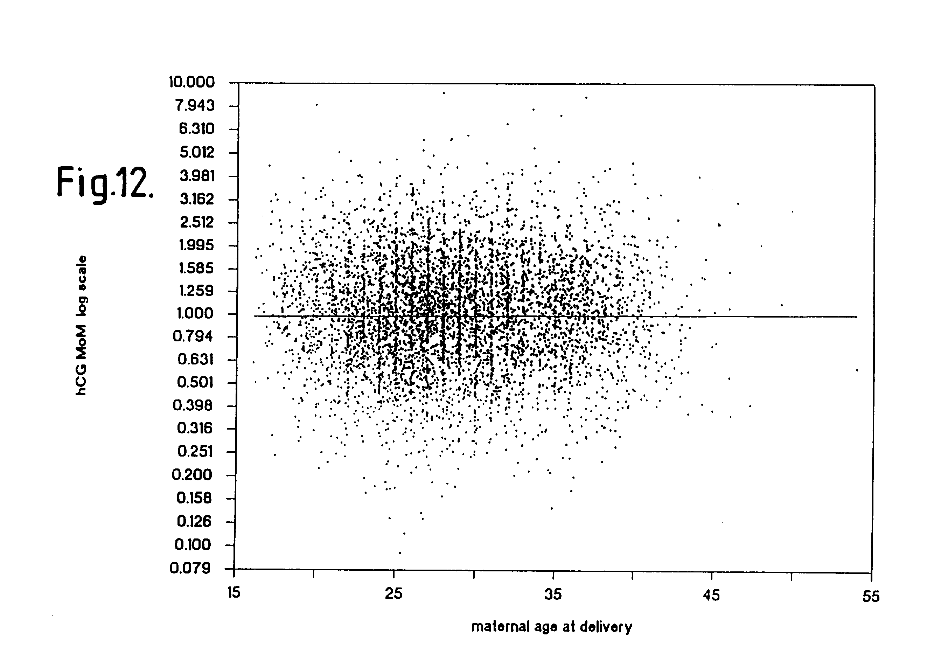

The relationship between maternal serum at the expected date of delivery and unconjugated estriol MoM for the combined data from Trials A and B is shown in Figure 10. The combined data for maternal serum AFP MoM is shown in Figure 11, and that for maternal serum hCG MoM in Figure 12.

Table 1 shows the correlation and regression between unconjugated estriol and maternal age at the expected date of delivery (EDD) for Trials A and B, and for both trials combined.

Table 1

| Regression of: log (UE MoM) = A * maternal age at EDD + B | | | |
|---|---|---|---|
| | Trials A | Trials B | combined |
| Constant B | 0.0536 | 0.0616 | 0.0553 |
| Std Err of Constant B | 0.3344 | 0.3448 | 0.3401 |
| 95% CI of constant B from | -0.6017 | -0.6141 | -0.6113 |
| to | 0.7090 | 0.7373 | 0.7218 |
| R Squared | 0.0022 | 0.0014 | 0.0018 |
| R value | 0.0470 | 0.0372 | 0.0420 |
| P value | $p < 0.01$ | $p < 0.05$ | $p < 0.01$ |
| No. of Observations | 2545 | 3124 | 5669 |
| Degrees of Freedom | 2543 | 3122 | 5667 |
| A Coefficients | -0.0025 | -0.0028 | -0.0026 |
| Std Err of A Coefficient | 0.0010 | 0.0014 | 0.0008 |
| 95% CI of A coefficient from | -0.0045 | -0.0055 | -0.0042 |
| to | -0.0004 | -0.0002 | -0.0010 |

Table 2 shows the lack of any statistically significant relationship between maternal serum AFP MoM and maternal age, and Table 3 similarly for maternal serum hCG and maternal age

Table 2

| Regression of: log (AFP MoM) = A * maternal age at EDD + B | | | |
|---|---|---|---|
| | Trials A | Trials B | combined |
| Constant B | -0.0274 | 0.0172 | -0.0017 |
| Std Err of Constant B | 0.3715 | 0.3873 | 0.3803 |
| 95% CI of constant B from | -0.7557 | -0.7420 | -0.7471 |
| to | 0.7008 | 0.7763 | 0.7436 |
| R Squared | 0.0002 | 0.0000 | 0.000 |
| R value | 0.0130 | 0.0050 | 0.005 |
| P value | n.s. | n.s. | n.s. |

Table 2   (continued)

| Regression of: log (AFP MoM) = A * maternal age at EDD + B | | | |
|---|---|---|---|
| | Trials A | Trials B | combined |
| No. of Observations | 2545 | 3124 | 5669 |
| Degrees of Freedom | 2543 | 3122 | 5667 |
| A Coefficients | 0.0008 | -0.0004 | 0.0001 |
| Std Err of A Coefficient | 0.0012 | 0.0015 | 0.0009 |
| 95% CI of A coefficient from | -0.0015 | -0.0034 | -0.0017 |
| to | 0.0030 | 0.0026 | 0.0019 |

Table 3

| Regression of: log (hCG MoM) = A * maternal age at EDD + B | | | |
|---|---|---|---|
| | Trials A | Trials B | combined |
| Constant B | 0.0101 | -0.0451 | -0.0260 |
| Std Err of Constant B | 0.5407 | 0.5549 | 0.5486 |
| 95% CI of constant B from | -1.0496 | -1.1327 | -1.1011 |
| to | 1.0698 | 1.0425 | 1.0492 |
| R Squared | 0.0000 | 0.0001 | 0.0000 |
| R value | 0.0034 | 0.0079 | 0.0058 |
| P value | n.s. | n.s. | n.s. |
| No. of Observations | 2545 | 3124 | 5669 |
| Degrees of Freedom | 2543 | 3122 | 5667 |
| A Coefficients | -0.0003 | 0.0010 | 0.0006 |
| Std Err of A Coefficient | 0.0017 | 0.0022 | 0.0013 |
| 95% CI of A coefficient from | -0.0036 | -0.0033 | -0.0020 |
| to | 0.0030 | 0.0053 | 0.0032 |

Example 2

Formula for adjusting UE MoM for maternal age.

From the combined data from trials A and B it can be seen that the corrected normal UE median MoM can be obtained by the following relationship:-

Correlated normal median MoM

$$= \exp(-0.0025758 * \text{age at EDD} + 0.55278)$$

The corrected MoM for an individual woman is then calculated as follows:-

Corrected MoM = Uncorrected MoM/Corrected normal median MoM

Table 4 shows the corrected MoM values for various combinations of maternal age at expected date of delivery and UE MoM values

Table 4

| Maternal age at EDD | uncorrected UE MoM | | |
|---|---|---|---|
| | 0.50 | 1.00 | 1.50 |
| | corrected UE MoM | | |
| 15 | 0.4917 | 0.9835 | 1.4752 |
| 20 | 0.4981 | 0.9962 | 1.4944 |
| 30 | 0.5111 | 1.0222 | 1.5334 |
| 40 | 0.5245 | 1.0489 | 1.5734 |
| 45 | 0.5313 | 1.0625 | 1.5938 |

Using the statistical distributions of maternal serum UE in unaffected Down Syndrome affected pregnancies given by Wald et al for pregnancies dated by ultrasound (Wald et al (1992) British Journal of Obstetrics and Gynaecology 99, 144-149) it is possible to calculate the effects that such corrections would have on the risks assigned to an individual woman if she were to be screened for Down Syndrome using as an example a combination of her age and maternal serum unconjugated estriol.

Table 5 shows the individual risks which would be assigned to women of different ages with and without such correction for the effect of maternal age on unconjugated estriol levels.

Table 5

| age at EDD | age risk | uncorrected UE MoM | risk assessment | |
|---|---|---|---|---|
| | | | uncorrected | corrected |
| 15 | 1:1578 | 0.40 | 1:21 | 1:24 |
| 15 | 1:1578 | 1.00 | 1:3134 | 1:3246 |
| 30 | 1:909 | 0.50 | 1:17 | 1:14 |
| 30 | 1:909 | 1.00 | 1:1877 | 1:1870 |
| 45 | 1:28 | 0.40 | 1:1 | 2:1 |
| 45 | 1:28 | 1.00 | 1:64 | 1.58 |

Correction for the influence of maternal age on unconjugated estriol levels has a direct influence on the assigned risk for fetal Down syndrome. A typical risk cut-off would be set at a risk of 1:250 as this approximately equals the risk of losing the baby through miscarriage as a result of the diagnostic amniocentesis. Depending upon the unconjugated estriol level and the mother's age the change could be sufficient for their risk to be altered from what would be considered high risk to low risk and vice-versa: some typical examples of these changes are shown in Table 6.

Table 6

| age at EDD | age risk | uncorrected UE MoM | risk assessment | |
|---|---|---|---|---|
| | | | uncorrected | corrected |
| 30 | 1:909 | 0.58 | 1:239 | 1:256 |
| 35 | 1:384 | 0.70 | 1:281 | 1:241 |
| 40 | 1:112 | 1.00 | 1:252 | 1:230 |

A second consequence of such a correlation is that it would be expected to lead to a slight reduction in the overall variance of UE MoM, and a consequential decrease in the numbers of women who screen-positive with such a test.

The changes illustrated have occurred when using maternal age and unconjugated estriol as a screening test. The changes in risk-assessment as a consequence of the relationship between maternal age and unconjugated estriol will still be present even if further analytes are added to the risk assessment, such as AFP, hCG, the free-beta or free alpha subunit of hCG, or any other maternal serum or fetal biometric marker for fetal abnormality.

Unconjugated estriol is also known to be a marker for trisomy 18 and for anencephaly and similar arguments apply for the inclusion of a maternal age correction factor to the UE MoM when screening for those conditions.

## Claims

1. A method for antenatal screening for chromosomal abnormalities in which a maternal body fluid from a pregnant woman is measured for the level of at least one marker and/or a precursor or metabolite of said marker and the measured level of this marker together with the gestational age of the pregnant woman are compared to reference values at various gestational ages of the level for the marker in (a) pregnant women carrying foetuses having the abnormalitie(s) subject to the screen and (b) pregnant women carrying normal foetuses, the comparison being indicative of the risk of the pregnant woman carrying a foetus with an abnormality subject to the screen characterised in that the level of the marker is affected by maternal age and, in making the comparison, allowance is made for the age of the pregnant woman.

2. A method according to claim 1 characterised in that a chromosomal abnormality which is screened for is Down Syndrome.

3. A method according to claim 1 or claim 2 characterised in that chromosomal abnormalities screened for include at least one of Edwards Syndrome (Trisomy 18), Pateaus Syndrome (Trisomy 13), Turner Syndrome, Monosomy X and Kleinefelter's Syndrome.

4. A method according to any one of the preceding claims characterised in that the marker to be measured is unconjugated estriol (UE).

5. A method according to any one of claims 1 to 3 characterised in that the marker to be measured is DHEAS or 16-alpha-hydroxy DHEAS.

6. A method according to any one of the preceding claims characterised in that measurement of an additional marker is included.

7. A method according to any one of the preceding claims characterised in that measurements are made on a sample of a body fluid taken in the period between the beginning of the eighth week and the end of the thirteenth week of gestation.

8. A method according to any one of the preceding claims characterised in that the maternal body fluid is blood and the marker is a serum marker.

9. An apparatus comprising means adapted for receiving measurements of a pregnant woman's maternal body fluid level of at least one marker and/or a precursor or metabolite of said marker and computer means for comparing the measurements of this level to sets of reference data to determine fetal chromosomal abnormalities characterised in that the level of the marker is affected by maternal age and the apparatus comprises means for adjusting the marker level measurements to take account of this.

10. An apparatus according to claim 9 comprising means adapted for receiving measurements of a pregnant woman's maternal blood levels of unconjugated estriol (UE).

## Patentansprüche

1. Verfahren zur vorgeburtlichen Überprüfung von Mißbildungen der Chromosomen, bei dem eine mütterliche Körperflüssigkeit von einer schwangeren Frau auf die Konzentration von zumindest einem Marker und/oder einem Vorläufer oder Metaboliten dieses Markers vermessen wird und die gemessene Konzentration dieses Markers zusammen mit dem Gestationsalter der schwangeren Frau mit Bezugswerten bei verschiedenen Gestationsaltern der Konzentration für den Marker bei

   (a) schwangeren Frauen, die Föten tragen mit der (den) Mißbildung(en), die Gegenstand der Überprüfung ist (sind), und
   (b) schwangeren Frauen, die normale Fölen tragen,

verglichen wird, wobei der Vergleich einen Hinweis auf das Risiko der schwangeren Frau gibt, daß sie einen Fötus mit einer Mißbildung trägt, die Gegenstand der Überprüfung ist, *dadurch gekennzeichnet, daß* die Konzentration des Markers durch mütterliches Alter beeinflußt wird, und daß beim Anstellen des Vergleichs das Alter der schwangeren Frau berücksichtigt wird.

2. Verfahren nach Anspruch 1, *dadurch gekennzeichnet, daß* eine chromosomale Mißbildung, auf die überprüft wird, Down-Syndrom ist.

3. Verfahren nach Anspruch 1 oder Anspruch 2, *dadurch gekennzeichnet, daß* die chromosomalen Mißbildungen, auf die überprüft wird, zumindest eines von Edwards-Syndrom (Trisomie 18), Pateaus-Syndrom (Trisomie 13), Turner-Syndrom, Monosomie X und Kleinefelter-Syndrom einschließen.

4. Verfahren nach einem der vorangehenden Ansprüche, *dadurch gekennzeichnet, daß* der zu messende Marker nicht-konjugiertes Östriol (UE) ist.

5. Verfahren nach einem der Ansprüche 1 bis 3, *dadurch gekennzeichnet, daß* der zu messende Marker DHEAS oder 16-alpha-Hydroxy-DHEAS ist.

6. Verfahren nach einem der vorangehenden Ansprüche, *dadurch gekennzeichnet, daß* Messung eines zusätzlichen Markers eingeschlossen ist.

7. Verfahren nach einem der vorangehenden Ansprüche, *dadurch gekennzeichnet, daß* Messungen an einer in der Periode zwischen dem Anfang der 8. Woche und dem Ende der 13. Woche der Schwangerschaft genommenen Probe von Körperflüssigkeit durchgeführt werden.

8. Verfahren nach einem der vorangehenden Ansprüche, *dadurch gekennzeichnet, daß* die mütterliche Körperflüssigkeit Blut ist und der Marker ein Serum-Marker ist.

9. Gerät, das Mittel, die dazu angepaßt sind, um Konzentrationsmessungen einer mütterlichen Körperflüssigkeit einer schwangeren Frau von zumindest einem Marker und/oder einem Vorläufer oder Metaboliten dieses Markers aufzunehmen, und Computer-Mittel umfaßt zum Vergleichen der Messungen dieser Konzentration mit Sätzen von Bezugsdaten, um fotale chromosomale Mißbildungen zu bestimmen, *dadurch gekennzeichnet, daß* die Konzentration des Markers durch mütterliches Alter beeinflußt wird und das Gerät Mittel zum Anpassen der Messungen der Markerkonzentration umfaßt, um dies zu berücksichtigen.

10. Gerät nach Anspruch 9, das Mittel umfaßt, die dazu angepaßt sind, um Messungen der Konzentrationen von nicht-konjugiertern Östriol (UE) du mütterlichen Blutes einer schwangeren Frau aufzunehmen.

## Revendications

1. Procédé pour le dépistage prénatal d'anomalies chromosomiques, dans lequel on détermine dans un liquide corporel maternel provenant d'une femme enceinte la concentration d'au moins un marqueur et/ou un précurseur ou métabolite dudit marqueur, et on compare la concentration trouvée de ce marqueur, conjointement avec l'age gestationnel de la femme enceinte, à des valeurs de référence à divers âges gestationnels de la concentration du marqueur chez (a) des femmes enceintes portant des foetus ayant l'anomalie (les anomalies) faisant l'objet du dépistage et (b) des femmes enceintes portant des foetus normaux, la comparaison étant indicatrice du risque que la femme enceinte porte un foetus ayant une anomalie faisant l'objet du dépistage, caractérisé en ce que la concentration du marqueur est affectée par l'âge maternel, et lorsqu'on fait la comparaison, on tient compte de l'age de la femme enceinte.

2. Procédé selon la revendication 1, caractérisé en ce qu'une anomalie chromosomique qui est dépistée est le syndrome de Down.

3. Procédé selon la revendication 1 ou 2, caractérisé en ce que les anomalies chromosomiques dépistées comprennent au moins l'un parmi le syndrome d'Edwards (trisomie 18), le syndrome de Patau (trisomie 13), le syndrome de Turner, la monosomie X et le syndrome de Klinefelter.

4. Procédé selon l'une quelconque des revendications précédentes, caractérisé en ce que le marqueur à doser est l'oestriol non conjugué (UE).

5. Procédé selon l'une quelconque des revendications 1 à 3, caractérisé en ce que le marqueur à doser est la DHEAS (déhydro-isoandrostérone) ou la 16-α-hydroxy-DHEAS.

6. Procédé selon l'une quelconque des revendications précédentes, caractérisé en ce qu'un dosage d'un marqueur supplémentaire est inclus.

7. Procédé selon l'une quelconque des revendications précédentes, caractérisé en ce que les dosages sont effectués sur un échantillon d'un liquide corporel prélevé dans la période entre le début de la huitième semaine et la fin de la treizième semaine de gestation.

8. Procédé selon l'une quelconque des revendications précédentes, caractérisé en ce que le liquide corporel maternel est du sang et le marqueur est un marqueur sérique.

9. Appareil, comprenant un moyen destiné à recevoir des mesures de la concentration, dans un liquide corporel maternel d'une femme enceinte, d'au moins un marqueur et/ou un précurseur ou métabolite dudit marqueur et un moyen informatique pour comparer les mesures de cette concentration à des séries de données de référence afin de déterminer des anomalies chromosomiques foetales, caractérisé en ce que la concentration du marqueur est affectée par l'age maternel, et l'appareil comprend un moyen pour ajuster les mesures de la concentration du marqueur afin d'en tenir compte.

10. Appareil selon la revendication 9, comprenant un moyen destiné à recevoir des mesures de concentrations, dans le sang maternel d'une femme enceinte, d'oestriol non conjugué (UE).

Fig.1.

Fig.2.

Fig. 3.

frequency

Normal outcome

Down syndrome

0.25

2.50

UE MoM (log scale)

Fig.4.

UE  MoM (log scale)

maternal age at date of delivery

EP 0 635 722 B1

13

Fig.5.

Fig.6.

maternal age at date of delivery

hCG MoM (log scale)

Fig.7.

maternal age at date of delivery

UE   MoM (log scale)

EP 0 635 722 B1

Fig.8.

Fig.9.

Fig.10.

UE    MoM. log scale

maternal age at delivery

Fig.11.

AFP MoM. log scale

maternal age at delivery

Fig.12.